# EUROPEAN PATENT SPECIFICATION

(11) **EP 4 641 485 B1**
(45) Date of publication and mention of the grant of the patent: **28.10.2026**
(21) Application number: 24172211.5
(22) Date of filing: 24.04.2024
(51) Int. Cl.: G06T 5/70

(54) **NOISE VARIANCE STABILIZATION IN X-RAY IMAGING**
RAUSCHVARIANZSTABILISIERUNG IN DER RÖNTGENBILDGEBUNG
STABILISATION DE LA VARIANCE DU BRUIT EN IMAGERIE PAR RAYONS X

(43) Date of publication of application: 29.10.2025
(73) Proprietor: Siemens Healthineers AG, 91301 Forchheim (DE)
(72) Inventor: Hariharan, Sai Gokul, 91301 Forchheim (DE); Leghissa, Martino, 91369 Wiesenthau (DE); Rohdjeß, Heiko, 91091 Grossenseebach (DE)
(74) Representative: Siemens Healthineers Patent Attorneys

(56) References cited:
- US-A1- 2021 019 863
- US-B2- 9 589 373
- HARIHARAN SAI GOKUL ET AL: "Robust learning-based x-ray image denoising-potential pitfalls, their analysis and solutions", BIOMEDICAL PHYSICS & ENGINEERING EXPRESS, vol. 8, no. 3, 7 April 2022 (2022-04-07), GB, pages 035013, XP093206252, ISSN: 2057-1976, DOI: 10.1088/2057-1976/ac3489
- SIEWERDSEN J H ET AL: "Empirical and theoretical investigation of the noise performance of indirect detection, active matrix flat-panel imagers (AMFPIs) for diagnostic radiology", MEDICAL PHYSICS, AIP, MELVILLE, NY, US, vol. 24, no. 1, 31 January 1997 (1997-01-31), pages 71, XP012010106, ISSN: 0094-2405, DOI: 10.1118/1.597919
- HARIHARAN SAI GOKUL ET AL: "Data-driven estimation of noise variance stabilization parameters for low-dose x-ray images", PHYSICS IN MEDICINE AND BIOLOGY, vol. 65, no. 22, 18 November 2020 (2020-11-18), Bristol GB, pages 1 - 16, XP093216296, ISSN: 0031-9155, DOI: 10.1088/1361-6560/abbc82
- GOEBEL P M ET AL: "Noise estimation in panoramic x-ray images: an application analysis approach", STATISTICAL SIGNAL PROCESSING, 2005 IEEE/SP 13TH WORKSHOP ON BORDEAUX, FRANCE JULY 17-20 2005, PISCATAWAY, NJ, USA,IEEE, 17 July 2005 (2005-07-17), pages 996 - 1001, XP010915042, ISBN: 978-0-7803-9403-2, DOI: 10.1109/SSP.2005.1628740

## Description

The present invention is directed to a computer-implemented method for generating a noise-variance-stabilized X-ray image, wherein an X-ray image generated by an X-ray imaging system is received, and to a computer-implemented method for processing an X-ray image, wherein a noise-variance-stabilized X-ray image is generated using said computer-implemented method. The invention is further directed to an X-ray imaging method, wherein an X-ray image is generated by an X-ray imaging system and said computer-implemented method is carried out, to a data processing system adapted to carry out said computer-implemented method, to an X-ray imaging system comprising such a data processing system, and to a corresponding computer program product.

In X-ray imaging, the noise content in 2D images depends on various parameters. One typical measure of image quality is the signal-to-noise ratio, SNR, which is determined as the ratio of the signal y in the image, which is for example expressed in digital units or so-called gray values, and which is provided by the X-ray detector, to the noise standard deviation σ. It is well known that SNR = y/σ depends on the X-ray dose, more specifically SNR scales typically with square root of the X-ray dose. However, improving the image quality by increasing the dose is limited by the ALARA ("As Low As Reasonably Achievable") principle, which basically requires to minimize or limit the X-ray dose.

A dose-independent quantity to characterize the noise content in X-ray images is the variance-to-signal ratio σ²/y. To be more precise, in flat panel X-ray imaging, the total noise variance may depend on various contributions, for example σ² = σ_{q}² + σₙ², with the quantum noise contribution σ_{q}²*,* which is related to the radiation field and is also referred to as Poisson noise, since it may be modelled using a Poisson distribution, and the electronic noise σₙ² of the X-ray detector, also referred as Gaussian noise, since it may be modelled using a Gaussian distribution.

Today's X-ray image processing pipelines need to work with the different noise characteristics, in particular different mixtures of Poisson and Gaussian noise, which can occur depending on various parameters of the imaging scenario. The different contributions depend on different imaging parameters of the X-ray imaging system, such as exposure parameters, geometry parameters, the imaged object, and so forth. Therefore, image processing algorithms, specially denoising algorithms but also algorithms for edge enhancement, contrast enhancement, segmentation, et cetera, must take into consideration all different noise characteristics. This may require that different variants of the image processing algorithms or parameterizations are provided for different regimes of the noise characteristics.

This problem is overcome by applying a variance-stabilizing transformation to the X-ray image, which stabilizes or normalizes the noise variance for example to a known constant, such that the image processing pipeline is applied to always the same normalized noise characteristics. The general Anscombe transform, GAT, is such a variance-stabilizing transformation.

In the publication S. Hariharan et al.: "Learning-based X-ray Image Denoising Utilizing Model-based Image Simulations"*, in* Shen, D., et al.: "Medical Image Computing and Computer Assisted Intervention - MICCAI 2019.", MICCAI 2019, Lecture Notes in Computer Science, vol. 11769, Springer, Ch*am,* the generalized Anscombe transform is applied to medical X-ray imaging. Furthermore, it is described how a trained artificial neural network can be used as a denoising algorithm based on accordingly transformed X-ray images.

The usage of said noise variance-stabilization comes along with the challenge, that for applying the transformation, knowledge of noise parameters is needed, especially knowledge of a noise level parameter α = (σ² - σₙ²) / yₘ and the electronic noise σₙ², wherein yₘ corresponds to the mean signal. While σₙ² can be obtained by calibrating the X-ray detector, α can be obtained in a data-driven approach by analyzing the input X-ray images as described in the publication S. Hariharan et al.: "Data-driven estimation of noise variance stabilization parameters for low- dose x-ray images", 2020, Phys. Med. Biol., 65, 225027*.* However, this requires additional time-critical computational efforts, which is a drawback not only but especially for real-time fluoroscopy applications. Furthermore, purely data-driven approaches may suffer from stability problems in scenarios where the X-ray images exhibit a large degree of structure or intensity variation.

The dependency of noise variance-stabilization from noise parameters is also known from the publication Hariharan Sai Gokul ET AL: "Robust learning-based x-ray image denoising-potential pitfalls, their analysis and solutions", Biomedical Physics & Engineering Express, vol. 8, no. 3, 7 April 2022 (2022-04-07), page 035013, XP093206252.

The publications I. Cunningham et al: "A spatial-frequency dependent quantum accounting diagram and detective quantum efficiency model of signal and noise propagation in cascaded imaging systems.", 1994, Med. Phys., 21: 417-427 and J. Siewerdsen et. al: "Empirical and theoretical investigation of the noise performance of indirect detection, active matrix flat-panel imagers (AMFPIs) for diagnostic radiology.", 1997, Med Phys., 24(1), 71-89*,* describe analytical models of imaging systems.

The publication J. Punnoose et al.: "Technical Note: spektr 3.0-A computational tool for x-ray spectrum modeling and analysis.", Med Phys., 2016, 43 (8), 4711 describes a computational toolkit, denoted as spektr 3.0, to calculate X-ray spectra based on a tungsten anode spectral model using an interpolating cubic splines algorithm (TASMICS).

The *publication* S. Agostinelli et al.: "Geant4-a simulation toolkit", Nuclear Instruments and Methods in Physics Research Section A: Accelerators, Spectrometers, Detectors and Associated Equipment, 506 (3): 250 describes Geant4, a platform for simulating the passage of particles through matter using Monte-Carlo methods.

Simulating x-ray images is known as well, e.g. from US 9,589,373 B2. The publication from GOEBEL P M ET AL: "Noise estimation in panoramic x-ray images: an application analysis approach" discloses the use of simulation for noise estimaton.

In the publication by O. Ronneberger et al.: "U-Net: Convolutional Networks for Biomedical Image Segmentation" (arXiv:1505.04597*),* the U-Net architecture is described, a widely used CNN architecture for image segmentation, which may, however, also be used for other computer vision tasks.

It is an objective of the present invention to overcome or reduce said drawbacks of data-driven approaches when computing a noise-variance-stabilized X-ray image, in particular to increase the robustness and/or reduce the computational effort.

This objective is achieved by the subject matter of the independent claim. Further implementations and preferred embodiments are subject matter of the dependent claims.

The invention is based on the idea to determine a noise level parameter by simulating an energy deposition of X-ray quanta on the X-ray detector.

The present invention is defined by the appended claims.

According to an aspect of the invention, a computer-implemented method for generating a noise-variance-stabilized X-ray image is provided. Therein, an X-ray image, which is or has been generated by an X-ray imaging system, and a corresponding set of imaging parameters of the X-ray imaging system are received. A noise level parameter is computed by simulating an energy deposition of X-ray quanta emitted by an X-ray source of the X-ray imaging system on an X-ray detector of the X-ray imaging system depending on the set of imaging parameters. The noise-variance-stabilized X-ray image is generated by applying a variance-stabilizing transformation, which depends on the noise level parameter, to the X-ray image.

Unless stated otherwise, all steps of the computer-implemented method may be performed by a data processing system, which comprises at least one data processing device. In particular, the at least one data processing device is configured or adapted to perform the steps of the computer-implemented method. For this purpose, the at least one data processing device may for example store a computer program comprising instructions which, when executed by the at least one data processing device, cause the at least one data processing device to execute the computer-implemented method. The expressions "data processing system" and "at least one data processing device" may be used interchangeably, here and in the following. This holds also for respective expressions derived therefrom.

In case the at least one data processing device comprises two or more data processing devices, certain steps carried out by the at least one data processing device may also be understood such that different data processing devices carry out different steps or different parts of a step. In particular, it is not required that each data processing device carries out the steps completely. In other words, carrying out the steps may be distributed amongst the two or more data processing devices.

From each implementation of the computer-implemented method, a respective implementation of a method for generating a noise-variance-stabilized X-ray image, which is not purely computer-implemented, is obtained by including respective steps of generating the X-ray image by the X-ray imaging system.

The X-ray image is, in particular, a two-dimensional X-ray protection image, which displays an object or a part of the object, in particular a patient or a body part of the patient, which is for example, located on a patient table of the X-ray imaging system. This object or the part of the object is denoted as image object in the following.

The set of imaging parameters is given by a respective set of imaging parameters, which have been used or were present or were applied when generating the X-ray image by means of the X-ray imaging system. The set of imaging parameters may comprise, in particular, any parameters or conditions, which affect or potentially affect the noise content, in particular, the quantum noise present in the X-ray image. This may include exposure parameters, geometrical parameters, detector parameters of the X-ray detector, parameters of the imaged object, and so forth.

The noise level parameter α is, in particular, a parameter of the noise level function, NLF, of the imaging process, which depends on quantum noise. In particular, denoting the variance of the total noise by σ² = σ_{q}² + σₑ², wherein σ_{q}² denotes the variance of the quantum noise and σₑ² denotes the variance of the electronic noise, the NLF may be denoted by σ^{z} = α·yₘ + σₑ², with α = (σ² - σₑ²) / yₘ. Therein, yₘ corresponds to the mean signal in the resulting X-ray image.

The variance-stabilizing transformation depends parametrically on the noise level parameter α. Therefore, determining α is required in order to apply the variance-stabilizing transformation to the X-ray image. According to the invention, this is achieved by simulating said energy deposition. This can be understood such that computing the noise level parameter includes simulating the energy deposition. The energy deposition may, for example, be quantified and, in particular, simulated as ratio of the, within a detector pixel, squared deposited energy's mean to the mean of the deposited energy, sometimes denoted as E2E-value: *̅E̅*̅²̅/*E̅*. The noise level parameter is then computed depending on the E2E-value, for example based on calibration measurements assuming a linear relation between the noise level parameter and the E2E-value: *α = α + b* · *E̅²̅*/*E̅.*

Simulating the energy deposition may involve one or more simulation steps, which simulate respective physical processes occurring in the complete imaging path from generating the X-rays by the X-ray source, the X-rays being transmitted, scattered, absorbed and/or attenuated by the imaged object and/or other objects in the beam path, and/or further processes occurring until a part of the X-rays reaches the surface of the X-ray detector. In other words, one or more physically motivated models are used in order to compute the noise level parameter, in contrast to using a data-driven approach or image analysis. Furthermore, also other physical processes involved in the conversion of the deposited energy into respective detector signals of the X-ray detector and eventually the generation of the X-ray image in terms of image pixels may be modeled or simulated. In addition, physical processes involved in the generation of detector signals, also image processing algorithms in the pipeline may have an impact on the NLF. Therefore, the model for the NLF may consider such algorithmic processes.

The noise-variance-stabilized X-ray image may then be used as a basis for various known X-ray image processing algorithms, such as denoising algorithms, edge enhancement algorithms, contrast enhancement algorithms, medical segmentation algorithms, and so forth. In particular, the noise-variance-stabilized X-ray image may be used for said algorithms instead of the X-ray image itself, which has the advantage that the input to the respective algorithm always has a consistent noise variance irrespective of the imaging parameters used for generating the respective X-ray image.

According to several implementations, a variance of an electronic noise of the X-ray detector is received and the variance stabilizing transformation is carried out depending on the variance of the electronic noise. In particular, the variance stabilizing transformation depends parametrically on the variance of the electronic noise.

In this way, the reliability of the variance stabilization is improved. The variance of the electronic noise like for example be obtained from calibration measurements of the X-ray detector. It can for example be considered as a given parameter of the X-ray detector and, in particular, its value does not need to be determined during the computer-implemented method according to the invention.

According to several implementations, required transformation parameters for all relevant different scenarios are precalculated and derived at runtime, for example using a lookup table. According to several implementations, the precalculated transformation parameters are parametrized as a function of the imaging parameters.

According to the invention, the noise level parameter is computed depending on the simulated energy deposition by using a detector model for the X-ray detector, which models a conversion of X-ray quanta to optical photons and a conversion of the optical photons to an electrical detector signal, in particular a digital electrical detector signal and, for example, a construction of respective pixel values of the X-ray image depending on the electrical detector signal.

In this way, the accuracy of the determined noise level parameter is further increased. Respective detector models for X-ray detectors are known, for example cascaded linear system models. In particular, detector models according to the publications of Cunningham et al. or Siewerdsen et al. mentioned in the introductory part of the present disclosure may be used.

According to such models, the noise level parameter may for example be computed as $α = b ⋅ \left({c}_{0}+{c}_{1}\frac{\overline{{E}^{2}}}{\overline{E}}\right) ,$

Wherein the parameters are determined by fitting respective experimental calibration data using the X-ray imaging system and, for example, phantom objects to obtain calibration data for various different sets of imaging parameters. However, alternatively or in addition, real patient measurements may be used.

According to the invention, the set of imaging parameters comprises exposure parameters of the X-ray source, the exposure parameters comrising a peak kilovoltage of the X-ray source used for generating the X-ray image and a tube current of the X-ray source used for generating the X-ray image and an X-ray pulse duration used for generating the X-ray image.

Exposure parameter like this are known to have a significant impact on the energy spectrum of the X-ray quanta emitted by the X-ray source. Consequently, they also affect the noise content in the X-ray image and, in particular, the variance of the quantum noise and the noise level parameter significantly. Simulating the energy deposition depending on the exposure parameters therefore allows to determine the noise level parameter with increased accuracy and, consequently, makes the variance stabilization more effective.

According to several implementations, the set of imaging parameters comprises filter properties of an X-ray filter of the X-ray imaging system, for example a filter material and/or a filter thickness of the X-ray filter.

The X-ray filter is, in particular, arranged between the X-ray source and the imaged object. The X-ray filter and the X-ray source may for example be part of a source unit of the X-ray imaging system, for example of a C-arm of the X-ray imaging system. The choice of the filter material may for example depend on an anode material of the X-ray source and on the targeted amount of beam hardening. A common choice for tungsten anodes are copper filters. However, others filter materials may include nickel, aluminum or other metals.

The filter material as well as the filter thickness have a significant impact on the energy spectrum of the X-ray quanta after passing the X-ray filter. Consequently, they also affect the noise content in the X-ray image and, in particular, the variance of the quantum noise and the noise level parameter significantly. Simulating the energy deposition depending on the filter material and/or the filter thickness therefore allows to determine the noise level parameter with increased accuracy and, consequently, makes the variance stabilization more effective.

According to several implementations, the set of imaging parameters comprises collimator properties of an X-ray collimator of the X-ray imaging system, for example a collimator opening size of the X-ray collimator.

The X-ray collimator is, in particular, arranged between the X-ray filter and the imaged object. The X-ray collimator may for example be part of the source unit of the X-ray imaging system. The choice of the collimator opening size may for example depend on the part of the object, which shall be imaged.

The collimator opening size has a significant impact for example on the scattering of X-ray quanta at the collimator and, since the scattering is energy dependent, also on the energy spectrum of the X-ray quanta after passing the X-ray filter. Consequently, it also affects the noise content in the X-ray image and, in particular, the variance of the quantum noise and the noise level parameter significantly. Simulating the energy deposition depending on the collimator properties therefore allows to determine the noise level parameter with increased accuracy and, consequently, makes the variance stabilization more effective.

According to several implementations, the set of imaging parameters comprises a zoom factor used for generating the X-ray image.

The zoom factor corresponds to a part of a detector array of the X-ray detector, whose data is used for generating the X-ray image, and which may be smaller than the full detector array. While the zoom factor may have a smaller effect on the noise content of the X-ray image than the exposure parameters, the filter properties and the collimator properties, the effect may not be neglectable in some applications. Simulating the energy deposition depending on the zoom factor may therefore make the variance stabilization more effective.

According to several implementations, the set of imaging parameters comprises geometrical parameters of the X-ray imaging system and the imaged object, respectively.

The geometrical parameters may for example comprise a position and/or an orientation of the X-ray source with respect to the imaged object and/or a position and/or orientation of the X-ray detector with respect to the imaged object. For example, in particular in case the X-ray imaging system comprises a C-arm carrying the X-ray source and the X-ray detector, the geometrical parameters may comprise a position and/or an angulation, in particular an angular position and an orbital position, of the C-arm.

The geometrical parameters may for example comprise, in some implementations, a position and/or an orientation of a patient table, on which the imaged object is located, or a part of the patient table.

The geometrical parameters may also comprise an effective thickness of the imaged object, for example in terms of a water equivalent thickness, WET. The geometrical parameters may also comprise an effective thickness of another object in the beam path between the X-ray source and the X-ray detector, for example the anti-scattering grid, a further anti-scattering grid, and/or a spectral filter.

The geometrical parameters have a significant impact for example on the scattering and transmission and, for example, absorption of X-ray quanta by the imaged object and, in some cases, further objects in the beam path. Consequently, they also affect the noise content in the X-ray image and, in particular, the variance of the quantum noise and the noise level parameter significantly. Simulating the energy deposition depending on the geometrical parameters therefore allows to determine the noise level parameter with increased accuracy and, consequently, makes the variance stabilization more effective.

According to several implementations, the set of imaging parameters comprises a gain factor of the X-ray detector and/or a status parameter of an anti-scattering grid of the X-ray imaging system and/or a pixel binning parameter of the X-ray detector. The status parameter of the anti-scattering grid may for example be whether the anti-scattering grid is in the beam path or not.

The gain factor, the pixel binning parameter and the presence of the anti-scattering grid in the beam path have a significant impact for example on the noise content in the X-ray image and, in particular, the variance of the quantum noise and the noise level parameter. Simulating the energy deposition depending on the gain factor and/or the status parameter of an anti-scattering grid and/or the pixel binning parameter therefore allows to determine the noise level parameter with increased accuracy and, consequently, makes the variance stabilization more effective.

According to several implementations, the set of imaging parameters comprises a status parameter of a further removable and/or configurable part in the beam path, which affect X-ray absorption. The explanations with regard to the anti-scattering grid hold analogously.

According to several implementations, the variance-stabilizing transformation is a generalized Anscombe transformation.

The generalized Anscombe transformation is on the one hand a well-known variance-stabilizing transformation, which is also applicable in the context of X-ray images, and, on the other hand, has proven particularly suitable for mixtures of Poisson and Gaussian noise. Consequently, a particularly reliable variance stabilization is achieved. The generalized Anscombe transformation is for example given by $y ′ = \frac{2}{α} \sqrt{α ⋅ y + \frac{3}{8} {α}^{2} + {σ}_{n}^{2}} ,$ wherein y denotes a pixel value of the X-ray image, y' denotes the respective pixel value of the noise-variance-stabilized X-ray image, and ${σ}_{n}^{2}$ denotes the variance of the electronic noise of the X-ray detector, which is predetermined, for example during a calibration phase. *α* denotes the noise level parameter computed by simulating the energy deposition as described.

Alternatively, other variance-stabilizing transformations of the form $y ′ = f \left(α\right) \sqrt{h ⋅ α + g \left(α, {σ}_{n}^{2}\right) ,}$ **may be used,** wherein f and g denote functions of *α* and (*α,* ${σ}_{n}^{2}$), respectively, and h is a constant.

According to several implementations, a Monte Carlo model of the X-ray imaging system and/or an analytical model of the X-ray imaging system is used to simulate the energy deposition.

For the Monte Carlo model, for example Geant4 may be used. The analytical model may for example be based on the formalism of Cunningham et al. mentioned above.

In this way, a particularly accurate simulation of the energy deposition and, and some implementations, further processes happening in the X-ray detector, may be achieved.

According to several implementations, the simulation of the energy deposition is performed offline and the result of the simulation is stored in a look-up-table, which is read-out in real-time.

According to several implementations, simulating the energy deposition includes simulating a collimation, in particular of X-ray beams or X-ray quanta, by an X-ray collimator of the X-ray imaging system, for example using one or more Monte Carlo simulations.

Consequently, a particularly accurate simulation of the energy deposition is achieved.

According to several implementations, simulating the energy deposition includes simulating an X-ray attenuation and/or X-ray scattering and/or X-ray transmission by the imaged object and/or by one or more further objects in the beam path, for example using one or more Monte Carlo simulations.

Consequently, a particularly accurate simulation of the energy deposition is achieved.

In particular, the effective thickness of the they imaged object may be used to simulate the attenuation, scattering and/or transmission of the imaged object.

According to a further aspect of the invention, a computer-implemented method for processing an X-ray image is provided. Therein, a noise-variance-stabilized X-ray image is generated using a computer-implemented method according to the invention. An image processing algorithm is applied to the noise-variance-stabilized X-ray image. As a result, a processed X-ray image is generated.

The image processing algorithm may, for example, comprise a denoising algorithm. In this case, the noise-variance-stabilization is particularly beneficial, since the same denoising algorithm can be used for different scenarios and, in particular imaging parameters, even though the noise content differs on the level of the X-ray images before the noise-variance-stabilization.

The image processing algorithm may, alternatively or in addition, comprise an edge enhancement algorithm and/or a contrast enhancement algorithm and/or a medical segmentation algorithm, etcetera.

According to several implementations, a back-transformed X-ray image is computed by applying an inverse of the variance-stabilizing transformation to the processed X-ray image.

According to several implementations, applying the image processing algorithm, for example the denoising algorithm, comprises applying a trained machine learning model, MLM, to the noise-variance-stabilized X-ray image.

In this case, the invention is particularly beneficial, since the MLM otherwise would have to be trained based on training images according to a huge variety of different imaging parameters and corresponding noise contents in order to be applicable universally. The MLM may for example be designed as described in the publication of Hariharan et al mentioned in the introductory part of the present disclosure.

In general terms, a trained MLM may mimic cognitive functions that humans associate with other human minds. In particular, by training based on training data the MLM may be able to adapt to new circumstances and to detect and extrapolate patterns. Another term for a trained MLM is "trained function".

In general, parameters of an MLM can be adapted or updated by means of training. In particular, supervised training, semi-supervised training, unsupervised training, reinforcement learning and/or active learning can be used. Furthermore, representation learning, also denoted as feature learning, can be used. In particular, the parameters of the MLMs can be adapted iteratively by several steps of training. In particular, within the training a certain loss function, also denoted as cost function, can be minimized. In particular, within the training of an artificial neural network, ANN, the backpropagation algorithm can be used.

In particular, an MLM can comprise an ANN, a support vector machine, a decision tree and/or a Bayesian network, and/or the MLM can be based on k-means clustering, Q-learning, genetic algorithms and/or association rules. In particular, an ANN can be or comprise a deep neural network, a convolutional neural network or a convolutional deep neural network. Furthermore, an ANN can be an adversarial network, a deep adversarial network and/or a generative adversarial network.

According to several implementations, the MLM is an ANN, in particular a convolutional neural network, CNN, for example a U-Net.

Such MLMs have proven as particularly image-to-image algorithms in the medical context.

According to several implementations, a detector model is calibrated to flat-field images acquired with the X-ray detector and the noise level parameter is computed depending on the detector model.

According to a further aspect of the invention, an X-ray imaging method is provided. Therein, an X-ray image is generated by an X-ray imaging system, in particular by an X-ray source and an X-ray detector of the X-ray imaging system, for example controlled by a control system of the X-ray imaging system. A computer-implemented method for processing the X-ray image according to the invention is carried out based on the X-ray image, for example by the control system and/or the data processing system, and the processed X-ray image or an image depending on the processed X-ray image, for example the back transformed X-ray image, is displayed by a display device, for example a display device of the X-ray imaging system.

According to several implementations, the X-ray imaging method is carried out as a fluoroscopy method, wherein a sequence of consecutive X-ray images including said X-ray image is generated by the X-ray imaging system and each of the consecutive X-ray images is processed by using a computer-implemented method for processing the X-ray image according to the inventio. Each processed X-ray image or an image depending on the respective processed X-ray image, is for example displayed by the display device.

The reduction of the computational effort by means of the computer-implemented method for generating a noise-variance-stabilized X-ray image according to the invention, is particularly beneficial in fluoroscopy methods, in particular in case the required computations are carried online or in real-time, at least partially.

According to several implementations, a Monte Carlo model of the X-ray imaging system is used to simulate the energy deposition.

According to several implementations, the simulation of the energy deposition is performed in real-time.

Further implementations of the X-ray imaging method according to the invention follow directly from the various embodiments of the computer-implemented methods according to the invention and vice versa. In particular, individual features and corresponding explanations as well as advantages relating to the various implementations of the computer-implemented methods according to the invention can be transferred analogously to corresponding implementations of the X-ray imaging method according to the invention.

According to a further aspect of the invention, a data processing system, which is adapted to carry out a computer-implemented method for generating a noise-variance-stabilized X-ray image according to the invention and/or a computer-implemented method for processing an X-ray image according to the invention, is provided.

In the present disclosure, the expressions "data processing system" and "at least one data processing device" may be used interchangeably. A data processing device may in particular be understood as a data processing device, which comprises processing circuitry. The data processing device can therefore in particular process data to perform computing operations. This may also include operations to perform indexed accesses to a data structure, for example a look-up table, LUT, as well as a data processing process implemented in hardware.

In particular, the data processing device may include one or more computers, one or more microcontrollers, and/or one or more integrated circuits, for example, one or more application-specific integrated circuits, ASIC, one or more field-programmable gate arrays, FPGA, and/or one or more systems on a chip, SoC. The data processing device may also include one or more processors, for example one or more microprocessors, one or more central processing units, CPU, one or more graphics processing units, GPU, and/or one or more signal processors, in particular one or more digital signal processors, DSP. The data processing device may also include a physical or a virtual cluster of computers or other of said units.

In various embodiments, the data processing device includes one or more hardware and/or software interfaces and/or one or more memory units.

A memory unit may be implemented as a volatile data memory, for example a dynamic random access memory, DRAM, or a static random access memory, SRAM, or as a non-volatile data memory, for example a read-only memory, ROM, a programmable read-only memory, PROM, an erasable programmable read-only memory, EPROM, an electrically erasable programmable read-only memory, EEPROM, a flash memory or flash EEPROM, a ferroelectric random access memory, FRAM, a magnetoresistive random access memory, MRAM, or a phase-change random access memory, PCRAM.

According to a further aspect of the invention, an X-ray imaging system is provided. The X-ray imaging system comprises an X-ray source, an X-ray detector, and a control system, which is configured to control the X-ray source and the X-ray detector to generate an X-ray image. The X-ray imaging system further comprises a data processing system, which is adapted to carry out a computer-implemented method for processing an X-ray image according to the invention.

The control system and the data processing system may be separated from each other. In this case, the control system may be understood to be or comprise a further data processing system according to the definition above. Alternatively, the data processing system of the X-ray imaging system comprises the control system. In particular, the control system may correspond to one or more data processing apparatuses of the data processing system.

For example, the X-ray imaging system comprises a display device, wherein the control system is configured to control the display device to display the processed X-ray image or an image depending on the processed X-ray image. In the latter case, the control system or the data processing system may be adapted to generate the image depending on the processed X-ray image. The processing may for example comprise applying one of one or more image processing algorithms mentioned above.

According to several implementations, the control system is configured to acquire flat-field images by controlling the X-ray source and the X-ray detector. The data processing system is configured to determine the respective noise level parameters of the acquired flat field images and to compare the noise level parameters of the acuired flat field images with the calculated noise level parameters as a quality check of for the X-ray imaging system.

Further implementations of the X-ray imaging system according to the invention follow directly from the various embodiments of the computer-implemented methods and the X-ray imaging method according to the invention and vice versa. In particular, individual features and corresponding explanations as well as advantages relating to the various implementations of the computer-implemented methods and the X-ray imaging method according to the invention can be transferred analogously to corresponding implementations of the X-ray imaging system according to the invention. In particular, the X-ray imaging system according to the invention is designed or programmed to carry out the X-ray imaging method according to the invention. In particular, the X-ray imaging system according to the invention carries out the X-ray imaging method according to the invention.

According to a further aspect of the invention, a computer program comprising instructions is provided. When the instructions are executed by a data processing system, the instructions cause the data processing system to carry out a computer-implemented method according to the invention.

The instructions may be provided as program code, for example. The program code can for example be provided as binary code or assembler and/or as source code of a programming language, for example C, and/or as program script, for example Python.

According to a further aspect of the invention, a further computer program comprising further instructions is provided. When the further instructions are executed by an X-ray imaging system according to the invention, in particular by the data processing system of the X-ray imaging system and/or the control system of the X-ray imaging system, the further instructions cause the X-ray imaging system to carry out an X-ray imaging method according to the invention.

The further instructions may be provided as program code, for example. The program code can for example be provided as binary code or assembler and/or as source code of a programming language, for example C, and/or as program script, for example Python.

According to a further aspect of the invention, a computer-readable storage medium storing a computer program and/or a further computer program according to the invention is provided.

The computer program, the further computer program and the computer-readable storage medium are respective computer program products comprising the instructions and/or the further instructions.

Further features and feature combinations of the invention are obtained from the figures and their description as well as the claims. In particular, further implementations of the invention may not necessarily contain all features of one of the claims. Further implementations of the invention may comprise features or combinations of features, which are not recited in the claims.

In the following, the invention will be explained in detail with reference to specific exemplary implementations and respective schematic drawings. In the drawings, identical or functionally identical elements may be denoted by the same reference signs. The description of identical or functionally identical elements is not necessarily repeated with respect to different figures.

In the figures,
- FIG 1: shows schematically an exemplary implementation of an X-ray imaging system according to the invention;
- FIG 2: shows a schematic flow diagram of an exemplary implementation of a computer-implemented method for generating a noise-variance-stabilized X-ray image according to the invention;
- FIG 3: shows a schematic flow diagram of an exemplary implementation of a computer-implemented method for processing an X-ray image according to the invention;
- FIG 4: shows a schematic flow diagram of applying a physics model in a further exemplary implementation of a computer-implemented method for generating a noise-variance-stabilized X-ray image according to the invention;
- FIG 5: shows schematically an X-ray source and an X-ray detector of a further exemplary implementation of an X-ray imaging system according to the invention as well as the imaged object and various processes that affect an X-ray beam in the beam path;
- FIG 6: shows a schematic flow diagram of a further exemplary implementation of a computer-implemented method for generating a noise-variance-stabilized X-ray image according to the invention;
- FIG 7: shows schematically a convolutional neural network;
- FIG 8: shows schematically an artificial neural network; and
- FIG 9: shows schematically a further convolutional neural network.

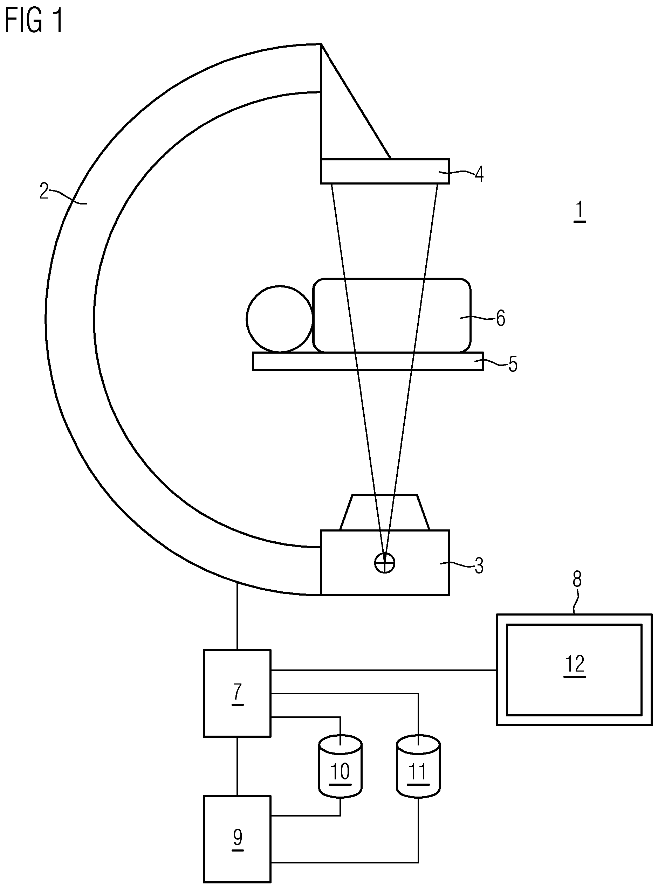

FIG 1 shows schematically an exemplary implementation of an X-ray imaging system 1 according to the invention. The X-ray imaging system 1 comprises a source unit 3 with an X-ray source, a detector unit 4 with an X-ray detector, and a control system 7, which is configured to control the X-ray source and the X-ray detector to generate X-ray images 13 depicting an object 6, for example a patient.

The X-ray imaging system 1 may for example comprise a patient table 5, on which the object 6 is arranged. The X-ray imaging system 1 comprises a data processing system 9 according to the invention, which is adapted to carry out a computer-implemented method for processing the X-ray image 13 according to the invention. In the following, several functions and method steps are described to be carried out by the control system 7, while other functions and method steps are described to be carried out by the data processing system 9. It is noted that the functions and method steps may also be distributed in different ways in alternative implementations.

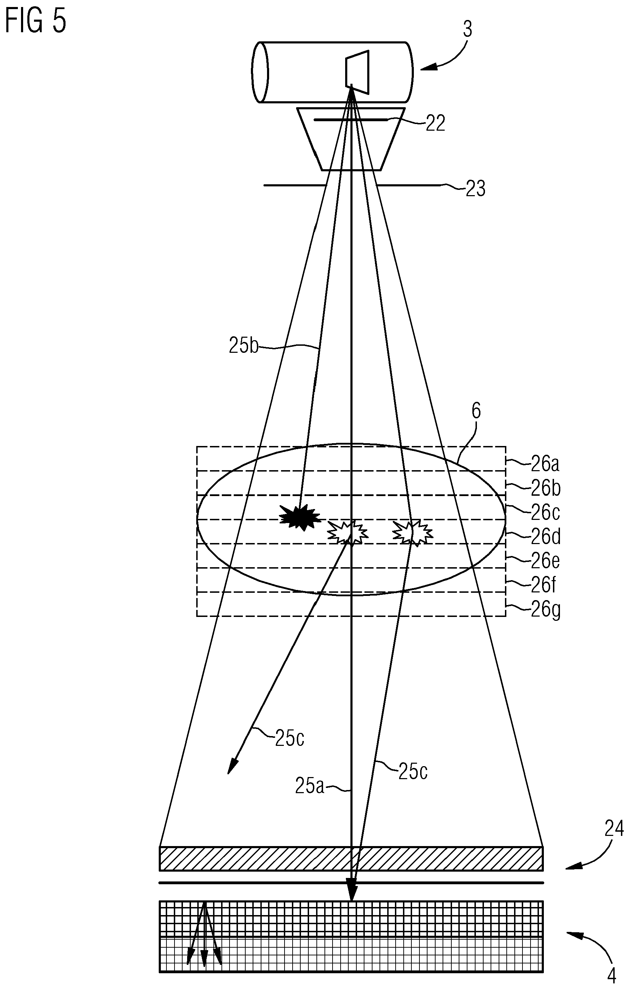

For example, the control system 7 may adjust various imaging parameters of the X-ray imaging system 1 including, for example, exposure parameters like a peak kilovoltage of the X-ray source, a tube current of the X-ray source, and/or an X-ray pulse duration. For example, the control system 7 may adjust further imaging parameters like a filter material and/or filter thickness of an X-ray filter 22, for example a copper filter, by placing the appropriate X-ray filter 22 into the beam path or by removing it from the beam path, respectively (see for example FIG 5). For example, the control system 7 may adjust further imaging parameters like a collimator opening size of an X-ray collimator 23. For example, the control system 7 may bring the X-ray collimator 23 into the beam path or by remove it from the beam path, respectively, (see for example FIG 5). For example, the control system 7 may adjust further imaging parameters like a gain factor of the X-ray detector. For example, the control system 7 may bring an anti-scattering grid 24 into the beam path or by remove it from the beam path, respectively, (see for example FIG 5). For example, the control system 7 may adjust a gain factor of the X-ray detector.

In some implementations, the X-ray imaging system 1 comprises a display device 8, wherein the control system 7 is configured to control the display device 8 to display the processed X-ray image 12 or an image depending on the processed X-ray image 12. In case a fluoroscopy-based medical intervention is carried out, the X-ray images 13 are for example generated as a sequence of consecutive frames to allow an operator to monitor or supervise the medical intervention. The X-ray imaging system 1 is, in particular, designed and adapted to carry out an X-ray imaging method according to the invention, wherein an X-ray image 13 is generated by the X-ray imaging system 1 using the X-ray source and the X-ray detector, and a computer-implemented method for processing the X-ray image 13 is carried out, in particular by the data processing system 9. The processed X-ray image 12 or the image depending on the processed X-ray image 12 is displayed on the display device 8.

In some implementations, the X-ray imaging system 1 is, as indicated in FIG 1, designed as a fluoroscopy system, in particular a C-arm fluoroscopy system, wherein the source unit 3 and the detector unit 4 are attached opposite to each other on a C-arm 2, which can be rotated around different axes. The corresponding motions are denoted as angular and orbital motion respectively. In some implementations, apart from the rotational motion of the C-arm 2, the patient table 5 and the C-arm 2 may be positioned by relative to each other by respective translatory motions of the C-arm 2 and/or the patient table 5. Consequently, the position and/or orientation of the X-ray source with respect to the object 6 and the position and/or orientation of the X-ray detector with respect to the object 6 may be adjusted exactly to the required imaging perspective.

In some implementations, the X-ray imaging system 1, for example the data processing system 9, may comprise databases 10, 11 to store and/or read data in the course of the computer-implemented method according to the invention and/or for other purposes.

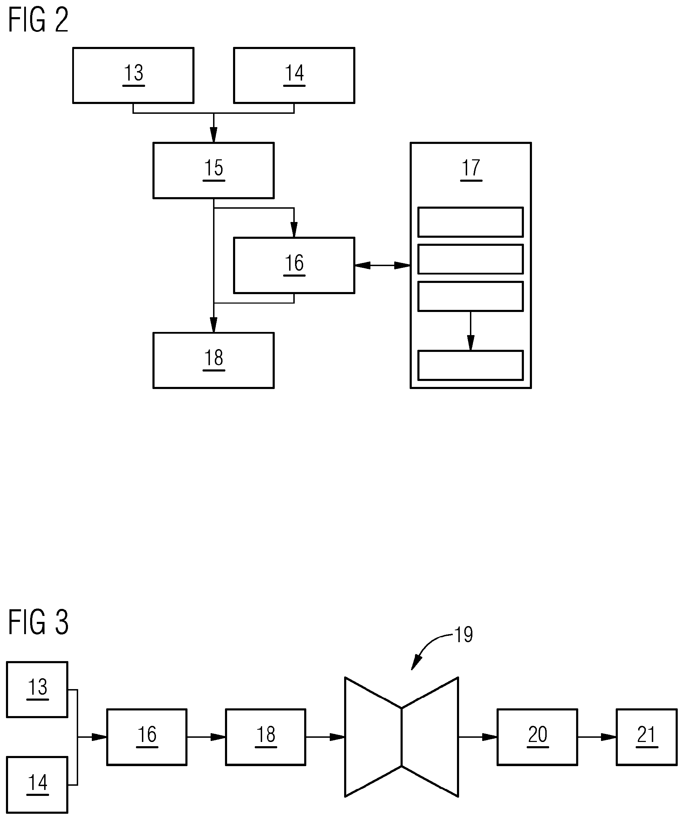

FIG 2 shows a schematic flow diagram of an exemplary implementation of a computer-implemented method for generating a noise-variance-stabilized X-ray image according to the invention.

Therein, the X-ray image 13 and a corresponding set of imaging parameters 14 of the X-ray imaging system 1 are received, for example by the data processing system 9, and, for example, fed to the imaging, processing pipeline 15. The imaging parameters 14 may comprise the peak kilovoltage of the X-ray source, the tube current of the X-ray source, and/or the X-ray pulse duration. A noise level parameter 16, for example the value α = (σ² - σₑ²) / yₘ, is computed by using a physics model 17 to, inter alia, simulate an energy deposition of X-ray quanta emitted by the X-ray source on the X-ray detector depending on the set of imaging parameters 14. A noise-variance-stabilized X-ray image 18 is generated by applying a variance-stabilizing transformation, in particular a general Anscombe transform, GAT, which depends on the noise level parameter, to the X-ray image 13. The GAT is for example computed according to $y ′ = \frac{2}{α} \sqrt{α ⋅ y + \frac{3}{8} {α}^{2} + {σ}_{n}^{2}} ,$ wherein y denotes a pixel value of the X-ray image 13, y' denotes the respective pixel value of the noise-variance-stabilized X-ray image 18, and ${σ}_{n}^{2}$ denotes the variance of the electronic noise of the X-ray detector, which is predetermined, for example during a calibration phase.

The noise level function in two-dimensional X-ray imaging is for example given by ${σ}^{2} = α ⋅ {y}_{m} + {{σ}_{e}}^{2} ,$ the first term denoting the variance of the quantum noise and the second term denoting the variance of the electronic noise. The variance of the quantum noise can, in principle, take any value, depends on the gray value yₘ (not corrupted by noise), and depends, via *α,* on the Imaging parameters 14, in particular the spectral distribution of X-ray quanta reaching the X-ray detector surface and also on the X-ray detector's properties. It is therefore relatively hard to estimate. Consequently, the noise level parameter *α*, is determined using the physics model 17, as described. On the other hand, the electronic noise essentially depends on the X-ray detector's properties only and can be determined by measurements for every individual detector mode.

The imaging parameters 14 may for example comprise the peak kilovoltage, the tube current, the X-ray pulse duration, the filter material and/or the filter thickness, the collimator opening size, the gain factor, and/or the presence or absence of the anti-scattering grid 24 in the beam path. The imaging parameters 14 may comprise the respective position and/or angulation, for example in terms of the angular angle and the orbital angle of the C-arm 2, of the X-ray source and the X-ray detector with respect to the object 6, and/or the position and/or orientation of the patient table 5. The imaging parameters 14 may comprise an effective thickness of the imaged object 6, for example in terms of its water-equivalent thickness, WET.

FIG 3 shows a schematic flow diagram of an exemplary implementation of a computer-implemented method for processing the X-ray image 13 according to the invention. Therein, the noise-variance-stabilized X-ray image 18 is generated, as described for example with respect to FIG 2.

Then, an image processing algorithm, for example an MLM 19 trained for denoising X-ray images, is applied to the noise-variance-stabilized X-ray image 18 and a back-transformed X-ray image 21 is generated by applying the inverse variance-stabilizing transformation to the output image 20 resulting as an output of the image processing algorithm. The back-transformed X-ray image 21 may also be denoted as processed X-ray image 21 and may be understood as a result of the method for processing the X-ray image 13.

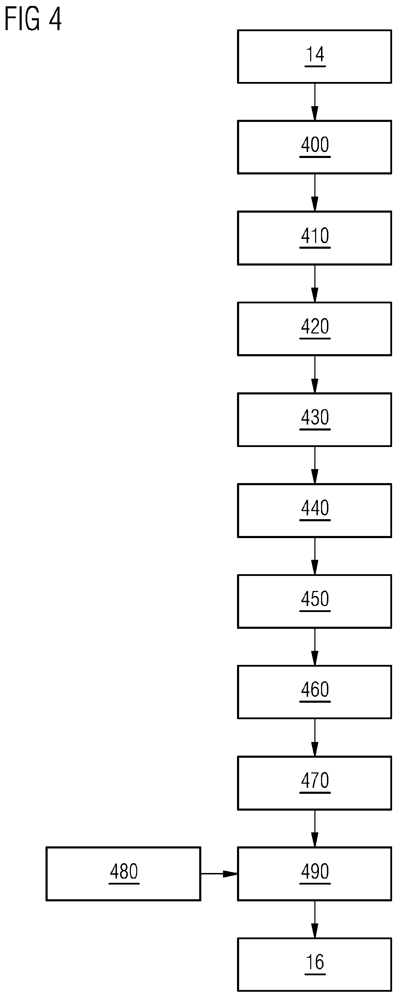

In the following, it is explained in more detail, how the physics model 17 is applied to compute the noise level parameter. FIG 4 shows a corresponding schematic flow diagram and FIG 5 shows schematically some simulated processes.

In step 400, the emission of X-ray quanta from the X-ray source, in particular X-ray tube, is simulated or estimated, for example using the computational toolkit spektr 3.0. The result is an initial energy spectrum. If the X-ray filter 22 is in the beam path, the change of the energy spectrum by the X-ray filter 22 is computed in step 410. Steps 400 and 410 can also be combined. Step 410 can also be carried out using spektr 3.0. In Step 420, the effect of the collimator 23 on the resulting energy spectrum is simulated, for example using a Monte Carlo simulation.

In step 430, the effect of an interaction, in particular scattering, absorption and transmission, of X-ray quanta with an additional object located between the X-ray source and the imaged object 6, in particular between the collimator 23 and the image object 6, on the resulting energy spectrum is simulated, in case such additional object is present. To this end, also a Monte Carlo simulation may be used. Depending on the position of the C-arm 2, the patient table 5 may for example be located between the X-ray source and the imaged object 6. In step 440 the effect of an interaction, in particular scattering, absorption and transmission, of X-ray quanta with the imaged object 6 itself on the resulting energy spectrum is simulated, in particular also by using one or more Monte Carlo simulation steps. In the example of FIG 5, the image object 6 is virtually divided into several slices 26a, 26b, 26c, 26d, 26e, 26f, 26g and a separate Monte Carlo simulation step is used to simulate the interaction in each slice 26a, 26b, 26c, 26d, 26e, 26f, 26g. For visualization purposes, X-ray quanta 25a, which are transmitted through the imaged object 6, X-ray quanta 25c, which are scattered in the imaged object 6, as well as X-ray quanta 25b, which are absorbed in the imaged object 6 are shown schematically.

In step 450, the effect of an interaction, in particular scattering, absorption and transmission, of X-ray quanta with an additional object located between the imaged object 6 and the X-ray detector 4, in particular between the image object 6 and the anti-scattering grid 24, on the resulting energy spectrum is simulated, in case such additional object is present. To this end, also a Monte Carlo simulation may be used. Depending on the position of the C-arm 2, the patient table 5 may for example be located between the imaged object 6 and the X-ray detector. In step 460, the effect of an interaction, in particular scattering, absorption and transmission, of X-ray quanta with the anti-scattering grid 24 on the resulting energy spectrum is simulated. To this end, also a Monte Carlo simulation may be used. In step 490 the energy deposition on the surface of the X-ray detector, in particular the E2E-value, is determined based on the finally resulting energy spectrum. To this end, also a Monte Carlo simulation may be used.

In step 490, the noise-variance parameter *α* is computed based on the energy deposition, in particular based on the E2E-value by using calibration measurements (step 480) and adapting them according to a suitable X-ray detector model, for example are cascaded linear systems model as for example the models of Cunningham at al. or Punnoose et al. mentioned in the introductory part of the present invention. In particular, the noise level parameter may be computed according to the equation $α = b ⋅ \left({c}_{0}+{c}_{1}\frac{\overline{{E}^{2}}}{\overline{E}}\right) ,$ **wherein** *b, c*₀ and *c*₁ are parameters obtained from the calibration measurements of step 480 and the E2E-value is for example computed as $\frac{\overline{{E}^{2}}}{\overline{E}} = \frac{{\int \left(1-exp\left(-{μ}_{c , pe}\left(E\right){t}_{c}\right)\right) {E}^{2} q}_{0} \left(E\right) dE}{{\int \left(1-exp\left(-{μ}_{c , pe}\left(E\right){t}_{c}\right)\right) E q}_{0} \left(E\right) dE} ,$ with *µ_{c,pe}* denoting the attenuation coefficient due to the photo-effect of the X-ray converter, in particular scintillator, of the X-ray detector, and *q*₀(*E*) is number of X-ray quanta per square-area per energy bin incident to the X-ray converter obtained from the last Monte Carlo simulation step. Using said model in step 490 effectively includes a conversion of X-ray quanta to optical photons, a conversion of the optical photons to detector signals, and a conversion of the detector signals to respective pixel signal information.

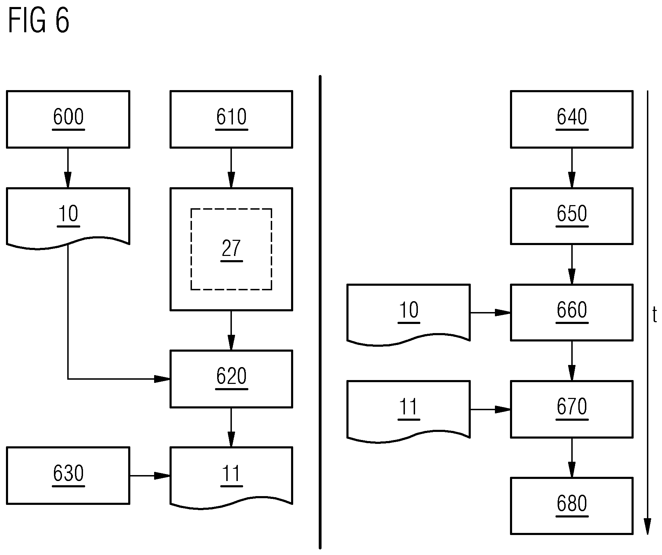

FIG 6 shows a schematic flow diagram of a further exemplary implementation of a computer-implemented method for generating a noise-variance-stabilized X-ray image according to the invention. In the shown implementation, the simulations and modelling as described for example with respect to FIG 5 are performed offline and the results are stored in the databases 10, 11 and implemented on the X-ray imaging system 1 via lookup tables and/or fit functions. For example, it is also possible to interpolate the data provided in the lookup tables according to the current requirements and system. Due to computational efforts and run time, especially of the Monte Carlo simulations, such implementations may be beneficial. However, in alternative implementations, the simulations and modeling are performed online in real-time and the results are transferred to the imaging pipeline in real-time.

For example, steps 600 to 630 of FIG 6 may be carried out by the data processing system 9, while the steps 640 to 680 may be carried out by the control system 7. However, this distribution may differ in other implementations. Step 600 of FIG 6 corresponds to steps 400 to 470 of FIG 4 resulting in the E2E-value, which is stored to the database 10. Step 610 of FIG 6 corresponds to step 480 of FIG 4, resulting in the respective calibration measurement data 27. These are, for example obtained by an analysis of multiple flat field images without imaging an object for many different values and combinations of the imaging parameters 14.

In step 620, the parameters *b, c*₀ and *c*₁ are obtained by fitting the calibration measurement data 27 to the equation for *α* above. In optional step 630, the influence of different image conditioning algorithms on the parameters *b, c*₀ and *c*₁ may be simulated. As a result, the various values for the parameters *b, c*₀ and *c*₁ may be stored to the database 11.

Steps 640 to 680 may, in particular, be carried out in real-time during the fluoroscopy. In step 640, the imaging parameters 14 concerning the X-ray source, the X-ray filter 22, the collimator 23 and the X-ray detector, are read for the current frame, for example the peak kilovoltage the tube current, the X-ray pulse duration, the filter material and/or the filter thickness, the collimator opening size, the gain factor, and/or the presence or absence of the anti-scattering grid 24 in the beam path. In step 640, the imaging parameters 14 concerning the geometric arrangement and the object 6, in particular position and/or angulation of the C-arm 2, the position and/or orientation of the patient table 5, and the WET, are read for the current frame. In step 660, the corresponding E2E-value is read from the database 10 for the current set of imaging parameters 14 and in step 670, the corresponding parameters *b, c*₀ and *c*₁ are read from the database 11 for the current set of imaging parameters 14. In step 680, the noise level parameter *α* is computed for the current frame depending on the read-out E2E-value and the read-out parameters *b, c*₀ and *c*₁ according to the equation above.

As described, according to the invention, a noise-variance-stabilized X-ray image 18 is obtained in a way that reduces the drawbacks of data-driven approaches.

In several implementations, the described method uses a physics model 17, which may be integrated into the X-ray imaging system 1, to estimate the NLF of the image chain, in particular the noise level parameter *α*. This leads to various advantages. For example, the determination of the NLF can be performed very fast and accurately and independent of the X-ray image 13 itself. The method is robust even in complex imaging scenarios where data-driven approaches may fail to determine the NLF reliably. The computational efforts and therefore computational hardware requirements can be relaxed leading to potential cost savings. This is also suitable for cost-efficient mobile C-arm systems.

In some embodiments, an implementation of the model-driven approach in parallel to a data-driven approach could provide a back-up or a possibility for consistency checks, thus reducing the occurrence of image artefacts due to non-optimal parameterization of image processing algorithms. An interchangeable implementation of data-driven and the model-based approach would allow to select either one of the two methods depending on the actual imaging situation.

Since the noise level parameter *α* is sensitive spectral changes of the X-ray source's output and X-ray detector's efficiency, the proposed method can, in some implementations, also be used to monitor and track the status of the X-ray imaging system 1, detect potential abnormal states, defects or degradation or aging of components and can also be used for system calibration purposes.

As the calculation of the Noise Level Function can be done independent or prior to acquiring the X-ray image 13, the method can, in some implementations, also be used to predict the NLF even before real images are acquired. This may help to improve X-ray exposure control methods.

In some implementations, also errors in the output of sensors, for example, the status of anti-scattering grid 23, and exposure parameters, may be predicted. This is possible by analyzing system parameters such as the peak kilovoltage, the filter thickness, the WET, the status of the anti-scattering grid 23, and the estimated *α*. In addition, a data-driven estimate can be used for improving the prediction of status of anti-scattering grid 23 and/or the WET, in some implementations. The status of anti-scattering grid 23 may help for performing a scatter correction later in the image processing pipeline, for example.

In some implementations, the evaluations can, for example, be done regularly as part of a calibration of the X-ray detector 4. Flat images, in particular without ab object to be imaged, may be acquired at specified exposure parameters, for example combinations of peak kilovoltage, rube current, and spectral filter. The values for *α* may be computed from variance and mean of the images. The value can be compared with the value estimated from the look up table. If there are deviations in the value, this may indicate a failure or malfunction of the X-ray source 3, the X-ray detector 4, the anti-scattering grid, and so forth. By acquiring several flat images at different spectra, it may for example be identified, in particular along with additional information from the achieved exposure parameters and WET estimation, whether the X-ray spectrum at the X-ray source 3 has changed or the response of the X-ray detector 4 has changed. Alternatively or in addition, damage of the anti-scattering grid may be predicted.

The MLM 19 depicted in FIG 3 may for example be an ANN.

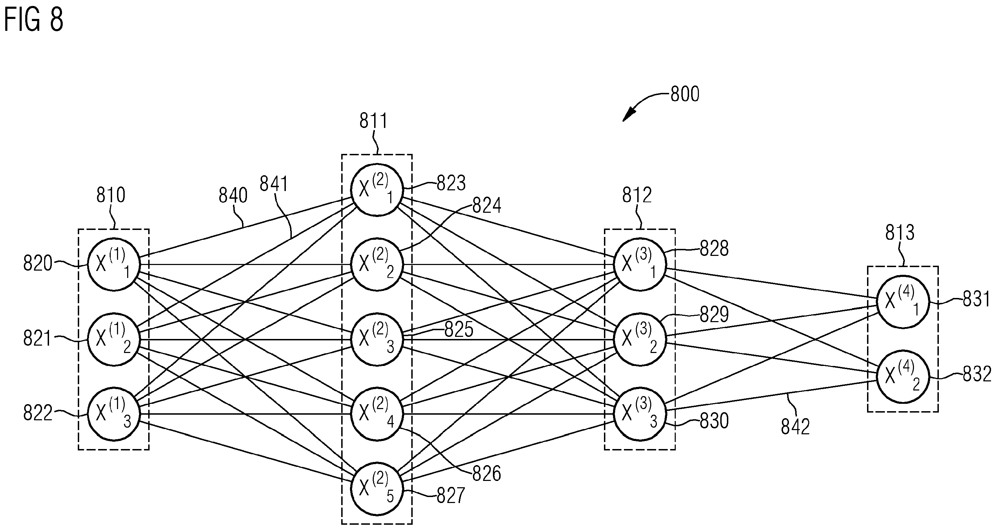

FIG 8 displays an embodiment of an ANN 800, which is for example designed as an MLP. The ANN 800 comprises nodes 820, ..., 832 and edges 840, ..., 842, wherein each edge 840, ..., 842 is a directed connection from a first node 820, ..., 832 to a second node 820, ..., 832. In general, the first node 820, ..., 832 and the second node 820, ..., 832 are different nodes 820, ..., 832. It is, however, also possible that the first node 820, ..., 832 and the second node 820, ..., 832 are identical. For example, in FIG 8, the edge 840 is a directed connection from the node 820 to the node 823, and the edge 842 is a directed connection from the node 830 to the node 832. An edge 840, ..., 842 from a first node 820, ..., 832 to a second node 820, ..., 832 is also denoted as ingoing edge for the second node 820, ..., 832 and as outgoing edge for the first node 820, ..., 832.

In this example, the nodes 820, ..., 832 of the artificial neural network 800 can be arranged in layers 810, ..., 813, wherein the layers can comprise an intrinsic order introduced by the edges 840, ..., 842 between the nodes 820, ..., 832. In particular, edges 840, ..., 842 can exist only between neighboring layers of nodes. In the displayed example, there is an input layer 810 comprising only nodes 820, ..., 822 without an incoming edge, an output layer 813 comprising only nodes 831, 832 without outgoing edges, and hidden layers 811, 812 in-between the input layer 810 and the output layer 813. In general, the number of hidden layers 811, 812 can be chosen arbitrarily. In an MLP, this number is at least one. The number of nodes 820, ..., 822 within the input layer 810 usually relates to the number of input values of the artificial neural network 800, and the number of nodes 831, 832 within the output layer 813 usually relates to the number of output values of the artificial neural network 800.

In particular, a real number can be assigned as a value to every node 820, ..., 832 of the artificial neural network 800. Here, x⁽ⁿ⁾ᵢ denotes the value of the i-th node 820, ..., 832 of the n-th layer 810, ..., 813. The values of the nodes 820, ..., 822 of the input layer 810 are equivalent to the input values of the artificial neural network 800. The values of the nodes 831, 832 of the output layer 813 are equivalent to the output value of the artificial neural network 800. Furthermore, each edge 840, ..., 842 can comprise a weight being a real number. In particular, the weight is a real number within the interval [-1, 1] or within the interval [0, 1]. Here, w^{(m,n)}_{i,j} denotes the weight of the edge between the i-th node 820, ..., 832 of the m-th layer 810, ..., 813 and the j-th node 820, ..., 832 of the n-th layer 810, ..., 813. Furthermore, the abbreviation w⁽ⁿ⁾_{i,j} is defined for the weight w^{(n,n+1)}_{i,j}. In particular, to calculate the output values of the neural network 800, the input values are propagated through the neural network 800. In particular, the values of the nodes 820, ..., 832 of the (n+1)-th layer 810, ..., 813 can be calculated based on the values of the nodes 820, ..., 832 of the n-th layer 810, ..., 813 by ${x}_{j}^{\left(n+1\right)} = f \left({\sum }_{i} {x}_{i}^{\left(n\right)} {w}_{i , j}^{\left(n\right)}\right) .$

Herein, the function f is denoted as transfer function or activation function. Known transfer functions are step functions, the sigmoid functions, for example the logistic function, the generalized logistic function, the hyperbolic tangent, the arctangent function, the error function, the smoothstep function, or rectifier functions. The transfer function is for example used for normalization purposes. In particular, the values are propagated layer-wise through the neural network 800, wherein values of the input layer 810 are given by the input of the neural network 800, wherein values of the first hidden layer 811 can be calculated based on the values of the input layer 810 of the neural network 800, wherein values of the second hidden layer 812 can be calculated based on the values of the first hidden layer 811, and so forth.

In order to set the values w^{(m,n)}_{i,j} for the edges, the neural network 800 has to be trained using training data. In particular, training data comprises training input data and training output data (denoted as tᵢ). For a training step, the neural network 800 is applied to the training input data to generate calculated output data. In particular, the training data and the calculated output data comprise a number of values, said number being equal with the number of nodes of the output layer. In particular, a comparison between the calculated output data and the training data is used to recursively adapt the weights within the neural network 800 (backpropagation algorithm). In particular, the weights are changed according to ${w}_{i , j}^{′ \left(n\right)} = {w}_{i , j}^{\left(n\right)} - γ {δ}_{j}^{\left(n\right)} {x}_{i}^{\left(n\right)} ,$ wherein γ is a predefined learning rate, and the numbers δ⁽ⁿ⁾ⱼ can be recursively calculated as ${δ}_{j}^{\left(n\right)} = \left({\sum }_{k} {δ}_{k}^{\left(n+1\right)} {w}_{j , k}^{\left(n+1\right)}\right) f ′ \left({x}_{i}^{\left(n\right)}{w}_{i , j}^{\left(n\right)}\right)$ based on δ⁽ⁿ⁺¹⁾ⱼ, if the (n+1)-th layer is not the output layer 813, and ${δ}_{j}^{\left(n\right)} = \left({x}_{j}^{\left(n+1\right)}-{t}_{j}^{\left(n+1\right)}\right) f ′ \left({x}_{i}^{\left(n\right)}{w}_{i , j}^{\left(n\right)}\right) ,$ if the (n+1)-th layer is the output layer 813, wherein f' is the first derivative of the activation function, and t⁽ⁿ⁺¹⁾ⱼ is the comparison training value for the j-th node of the output layer 813.

The MLM 19 may for example be a CNN. A convolutional neural network, CNN, is an ANN that uses a convolution operation instead of general matrix multiplication in at least one of its layers. These layers are denoted as convolutional layers. In particular, a convolutional layer performs a dot product of one or more convolution kernels with the convolutional layer's input data, wherein the entries of the one or more convolution kernel are parameters or weights that may be adapted by training. In particular, one can use the Frobenius inner product and the ReLU activation function. A convolutional neural network can comprise additional layers, for example pooling layers, fully connected layers, and/or normalization layers.

By using convolutional neural networks, the input can be processed in a very efficient way because a convolution operation based on different kernels can extract various image features so that by adapting the weights of the convolution kernel the relevant image features can be found during training. Furthermore, based on the weight-sharing in the convolutional kernels fewer parameters need to be trained, which prevents overfitting in the training phase and allows to have faster training or more layers in the network, improving the performance of the network.

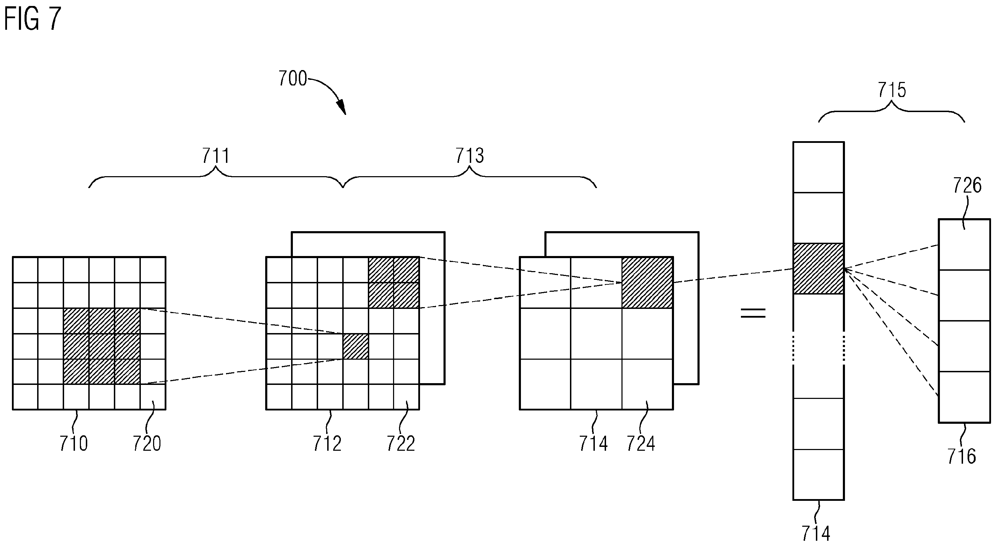

FIG 7 displays an exemplary embodiment of a convolutional neural network 700. In the displayed embodiment, the convolutional neural network 700 comprises an input node layer 710, a convolutional layer 711, a pooling layer 713, a fully connected layer 714 and an output node layer 716, as well as hidden node layers 712, 714. Alternatively, the convolutional neural network 200 can comprise several convolutional layers 711, several pooling layers 713 and/or several fully connected layers 715, as well as other types of layers. The order of the layers can be chosen arbitrarily, usually fully connected layers 715 are used as the last layers before the output layer 716.

In particular, within a convolutional neural network 700 nodes 720, 722, 724 of a node layer 710, 712, 714 can be considered to be arranged as a d-dimensional matrix or as a d-dimensional image. In particular, in the two-dimensional case the value of the node 720, 722, 724 indexed with i and j in the n-th node layer 710, 712, 714 can be denoted as x(n)[i, j]. However, the arrangement of the nodes 720, 722, 724 of one node layer 710, 712, 714 does not have an effect on the calculations executed within the convolutional neural network 700 as such, since these are given solely by the structure and the weights of the edges.

A convolutional layer 711 is a connection layer between an anterior node layer 710 with node values x(n-1) and a posterior node layer 712 with node values x(n). In particular, a convolutional layer 711 is characterized by the structure and the weights of the incoming edges forming a convolution operation based on a certain number of kernels. In particular, the structure and the weights of the edges of the convolutional layer 711 are chosen such that the values x(n) of the nodes 722 of the posterior node layer 712 are calculated as a convolution x(n) = K * x(n-1) based on the values x(n-1) of the nodes 720 anterior node layer 710, where the convolution * is defined in the two-dimensional case as ${x}^{\left(n\right)} \left[i, j\right] = \left(K∗{x}^{\left(n-1\right)}\right) \left[i, j\right] = {\sum }_{i ′} {\sum }_{j ′} K \left[i′,j′\right] ⋅ {x}^{\left(n-1\right)} \left[i-i′,j-j′\right] .$

Herein, the kernel K is a d-dimensional matrix, in the present example a two-dimensional matrix, which is usually small compared to the number of nodes 720, 722, for example a 3x3 matrix, or a 5x5 matrix. In particular, this implies that the weights of the edges in the convolution layer 711 are not independent, but chosen such that they produce said convolution equation. In particular, for a kernel being a 3x3 matrix, there are only 9 independent weights, each entry of the kernel matrix corresponding to one independent weight, irrespectively of the number of nodes 720, 722 in the anterior node layer 710 and the posterior node layer 712.

In general, convolutional neural networks 700 use node layers 710, 712, 714 with a plurality of channels, in particular, due to the use of a plurality of kernels in convolutional layers 711. In those cases, the node layers can be considered as (d+1)-dimensional matrices, the first dimension indexing the channels. The action of a convolutional layer 711 is then in a two-dimensional example defined as ${x}_{b}^{\left(n\right)} \left[i, j\right] = {\sum }_{a} \left({K}_{a , b}∗{x}_{a}^{\left(n-1\right)}\left[i, j\right]\right. = {\sum }_{a} {\sum }_{i ′} {\sum }_{j ′} {K}_{a , b} \left[i′,j′\right] ⋅ {x}_{a}^{\left(n-1\right)} \left[i-i′,j-j′\right] ,$ wherein ${x}_{a}^{\left(n\right)}$ corresponds to the a-th channel of the anterior node layer 710, ${x}_{b}^{\left(n\right)}$ corresponds to the b-th channel of the posterior node layer 712 and *K_{a,b}* corresponds to one of the kernels. If a convolutional layer 711 acts on an anterior node layer 710 with A channels and outputs a posterior node layer 712 with B channels, there are A·B independent d-dimensional kernels *K_{a,b}.*

In general, in convolutional neural networks 700 activation functions may be used. In this embodiment, ReLU (rectified linear unit) is used, with R(z) = max(0, z), so that the action of the convolutional layer 711 in the two-dimensional example is ${x}_{b}^{\left(n\right)} \left[i, j\right] = R \left({\sum }_{a} \left({K}_{a , b}∗{x}_{a}^{\left(n-1\right)}\left[i, j\right]\right.\right) = R \left({\sum }_{a} {\sum }_{i ′} {\sum }_{j ′} {K}_{a , b} \left[i′,j′\right] ⋅ {x}_{a}^{\left(n-1\right)} \left[i-i′,j-j′\right]\right) .$

It is also possible to use other activation functions, for example ELU (exponential linear unit), LeakyReLU, Sigmoid, Tanh or Softmax.

In the displayed embodiment, the input layer 710 comprises 36 nodes 720, arranged as a two-dimensional 6x6 matrix. The first hidden node layer 712 comprises 72 nodes 722, arranged as two two-dimensional 6x6 matrices, each of the two matrices being the result of a convolution of the values of the input layer with a 3x3 kernel within the convolutional layer 711. Equivalently, the nodes 722 of the first hidden node layer 712 can be interpreted as arranged as a three-dimensional 2x6x6 matrix, wherein the first dimension correspond to the channel dimension.

An advantage of using convolutional layers 711 is that spatially local correlation of the input data can exploited by enforcing a local connectivity pattern between nodes of adjacent layers, in particular by each node being connected to only a small region of the nodes of the preceding layer.

A pooling layer 713 is a connection layer between an anterior node layer 712 with node values x(n-1) and a posterior node layer 714 with node values x(n). In particular, a pooling layer 713 can be characterized by the structure and the weights of the edges and the activation function forming a pooling operation based on a non-linear pooling function f. For example, in the two-dimensional case the values x(n) of the nodes 724 of the posterior node layer 714 can be calculated based on the values x(n-1) of the nodes 722 of the anterior node layer 712 as ${x}_{b}^{\left(n\right)} \left[i, j\right] = f \left({x}_{b}^{\left(n-1\right)}\left[{id}_{1}, {jd}_{2}\right],…, {x}_{b}^{\left(n-1\right)}\left[\left(i+1\right){d}_{1}-1,\left(j+1\right){d}_{2}-1\right]\right) .$

In other words, by using a pooling layer 713, the number of nodes 722, 724 can be reduced by re-placing a number d1·d2 of neighboring nodes 722 in the anterior node layer 712 with a single node 722 in the posterior node layer 714 being calculated as a function of the values of said number of neighboring nodes. In particular, the pooling function f can be the max-function, the average or the L2-Norm. In particular, for a pooling layer 713 the weights of the incoming edges are fixed and are not modified by training.

The advantage of using a pooling layer 713 is that the number of nodes 722, 724 and the number of parameters is reduced. This leads to the amount of computation in the network being reduced and to a control of overfitting.

In the displayed embodiment, the pooling layer 713 is a max-pooling layer, replacing four neighboring nodes with only one node, the value being the maximum of the values of the four neighboring nodes. The max-pooling is applied to each d-dimensional matrix of the previous layer. In this embodiment, the max-pooling is applied to each of the two two-dimensional matrices, reducing the number of nodes from 72 to 18.

In general, the last layers of a convolutional neural network 700 may be fully connected layers 715. A fully connected layer 715 is a connection layer between an anterior node layer 714 and a posterior node layer 716. A fully connected layer 713 can be characterized by the fact that a majority, in particular, all edges between nodes 714 of the anterior node layer 714 and the nodes 716 of the posterior node layer are present, and wherein the weight of each of these edges can be adjusted individually.

In this embodiment, the nodes 724 of the anterior node layer 714 of the fully connected layer 715 are displayed both as two-dimensional matrices, and additionally as non-related nodes, indicated as a line of nodes, wherein the number of nodes was reduced for a better presentability. This operation is also denoted as flattening. In this embodiment, the number of nodes 726 in the posterior node layer 716 of the fully connected layer 715 smaller than the number of nodes 724 in the anterior node layer 714. Alternatively, the number of nodes 726 can be equal or larger.

Furthermore, in this embodiment the Softmax activation function is used within the fully connected layer 715. By applying the Softmax function, the sum the values of all nodes 726 of the output layer 716 is 1, and all values of all nodes 726 of the output layer 716 are real numbers between 0 and 1. In particular, if using the convolutional neural network 700 for categorizing input data, the values of the output layer 716 can be interpreted as the probability of the input data falling into one of the different categories.

In particular, convolutional neural networks 700 can be trained based on the backpropagation algorithm. For preventing overfitting, methods of regularization can be used, for example dropout of nodes 720, ..., 724, stochastic pooling, use of artificial data, weight decay based on the L1 or the L2 norm, or max norm constraints.

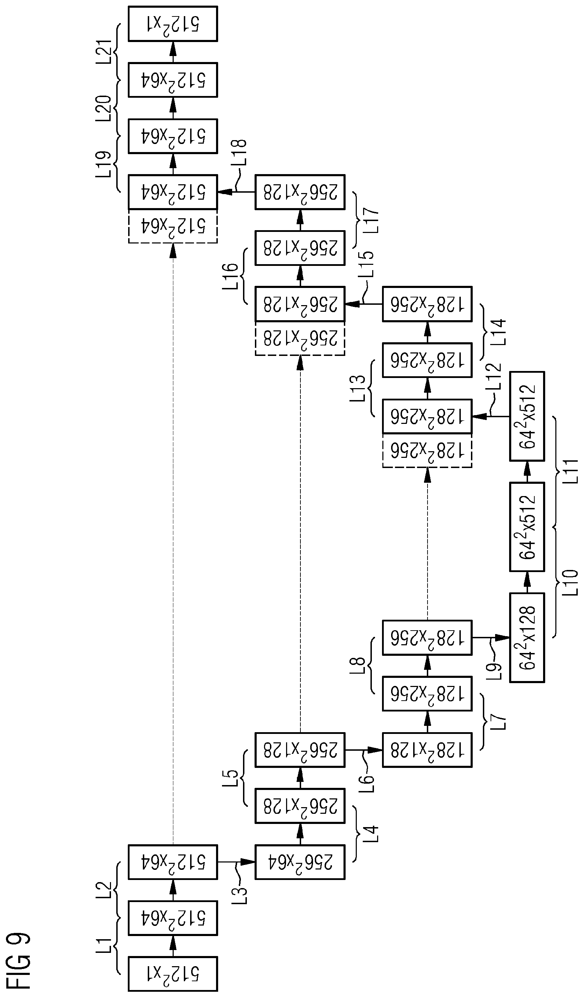

In the example of FIG 9, the MLM is a CNN, in particular, a convolutional neural network having a U-Net structure. In the displayed example, the input data to the CNN is a two-dimensional medical image comprising 512x512 pixels, every pixel comprising one intensity value. The CNN comprises convolutional layers indicated by solid, horizontal arrows, pooling layers indicating by solid arrows pointing down, and upsampling layers indicated by solid arrows pointing up. The number of the respective nodes is indicated within the boxes. Within the U-Net structure first the input images are downsampled, in particular by decreasing the size of the images and increasing the number of channels. Afterwards they are upsampled, in particular by increasing the size of the images and decreasing the number of channels, to generate a transformed image.

All except the last convolutional layers L1, L2, L4, L5, L.7, L8, L10, L11, L13, L14, L16, L17, L19, L20 use 3x3 kernels with a padding of 1, the ReLU activation function, and a number of filters or convolutional kernels that matches the number of channels of the respective node layers as indicated in FIG 9. The last convolutional layer uses a 1x1 kernel with no padding and the ReLU activation function.

The pooling layers L3, L6, L9 are max-pooling layers, replacing four neighboring nodes with only one node, the value being the maximum of the values of the four neighboring nodes. The upsampling layers L12, L15, L18 are transposed convolution layers with 3x3 kernels and stride 2, which effectively quadruple the number of nodes. The dashed horizontal arrows correspond to concatenation operations, where the output of a convolutional layer L2, L5, L8 of the downsampling branch of the U-Net structure is used as additional inputs for a convolutional layer L13, L16, L19 of the upsampling branch of the U-Net structure. This additional input data is treated as additional channels in the input node layer for the convolutional layer L13, L16, L19 of the upsampling branch.

For training the CNN, a database of 500 first medical images was used, wherein the respective segmentation mask was created based on annotations of expert radiologists. In particular, the experts determined for each of the 500 first medical images a segmentation mask for a structure of interest, where a value of 1 was assigned to pixels corresponding to the structure of interest, and a value of 0 was assigned to pixels not corresponding to the structure of interest. The database was split into training data (320 datasets), validation data (80 datasets) and test data (100 datasets). For training the CNN, the backpropagation algorithm was used based on a binary cross-entropy cost function $L \left(x, y\right) = {\sum }_{i} {\sum }_{j} BCE \left(y\left[i, j\right],M\left(x\right)\left[i, j\right]\right)$ with $BCE \left(a, b\right) : = - a log \left(b\right) \left(b\right) - \left(1-a\right) log \left(1-b\right) ,$ wherein x denotes a first medical image, y determines the corresponding segmentation mask created by the expert radiologist, and M(x) denotes the result of applying the CNN to the first input medical image x. Alternatively, one could use other cost functions like weighted binary cross entropy, Focal Loss or Dice Loss.

Based on the validation set of 80 datasets and the corresponding annotations, the best performing machine learning model out of several machine learning models (with different hyperparameters, for example number of layers, size and number of kernels, padding et cetera) was selected. The specificity and the sensitivity were determined based on the test set comprising 100 datasets and the corresponding annotations.

Independent of the grammatical term usage, individuals with male, female or other gender identities are included within the term.

## Claims

1. Computer-implemented method for generating a noise-variance-stabilized X-ray image (18), wherein
- an X-ray image (13) generated by an X-ray imaging system (1) and a corresponding set of imaging parameters (14) of the X-ray imaging system (1) are received, wherein the set of imaging parameters (14) comprises
- a peak kilovoltage of the X-ray source (3); and
- a tube current of the X-ray source (3); and
- an X-ray pulse duration;
**characterized in that**
- a noise level parameter (16) is computed by simulating an energy deposition of X-ray quanta emitted by an X-ray source (3) of the X-ray imaging system (1) on an X-ray detector (4) of the X-ray imaging system (1) depending on the set of imaging parameters (14),
wherein the noise level parameter (16) is computed depending on the simulated energy deposition by using a detector model for the X-ray detector (4), which models a conversion of X-ray quanta to optical photons and a conversion of the optical photons to an electrical detector signal; and
- the noise-variance-stabilized X-ray image (18) is generated by applying a variance-stabilizing transformation, which depends on the noise level parameter, to the X-ray image (13).

2. Computer-implemented method according to the preceding claim, wherein the set of imaging parameters (14) comprises
- a filter material and filter thickness of an X-ray filter (22) of the X-ray imaging system (1); or
- a collimator opening size of an X-ray collimator (23) of the X-ray imaging system (1); or
- a filter material and filter thickness of an X-ray filter (22) of the X-ray imaging system (1) and a collimator opening size of an X-ray collimator (23) of the X-ray imaging system (1).

3. Computer-implemented method according to one of the preceding claims, wherein the set of imaging parameters (14) comprises
- a position or orientation of the X-ray source (3); and
- a position or orientation of the X-ray detector (4); or
- a position or angulation of a C-arm (2) carrying the X-ray source (3) and the X-ray detector (4); and/or
- a position and/or orientation of a patient table (5) or a part of the patient table (5); and/or
- an effective thickness of an imaged object (6).

4. Computer-implemented method according to one of the preceding claims, wherein the set of imaging parameters (14) comprises
- a gain factor of the X-ray detector (4); or
- a status parameter of an anti-scattering grid (24) of the X-ray imaging system (1); or
- a pixel binning parameter of the X-ray detector (4).

5. Computer-implemented method according to one of the preceding claims, wherein the variance-stabilizing transformation is a generalized Anscombe transformation.

6. Computer-implemented method according to one of the preceding claims, wherein a Monte Carlo simulation is used or a sequence of Monte Carlo simulations are used to simulate the energy deposition, wherein the simulation of the energy deposition is performed offline and the result of the simulation is stored in a look-up-table, which is read-out in real-time.

7. Computer-implemented method according to one of the preceding claims, wherein simulating the energy deposition includes
- simulating a collimation by an X-ray collimator of the X-ray imaging system (1); or
- simulating an X-ray attenuation or X-ray scattering or X-ray transmission by the imaged object (6) or one or more further objects.

8. Computer-implemented method for processing an X-ray image (13), wherein a noise-variance-stabilized X-ray image (18) is generated using a computer-implemented method according to one of the preceding claims and
- a denoising algorithm (19) is applied to the noise-variance-stabilized X-ray image (18); or
- an image processing algorithm is applied to the noise-variance-stabilized X-ray image (18).

9. Computer-implemented method according to claim 8, wherein applying the denoising algorithm or the image processing algorithm comprises applying a trained machine learning model (19) to the noise-variance-stabilized X-ray image (18).

10. X-ray imaging method, wherein an X-ray image (13) is generated by an X-ray imaging system (1), and a computer-implemented method according to one of claims 8 or 9 is carried out based on the X-ray image (13), and the processed X-ray image (12, 21) or an image depending on the processed X-ray image (12, 21) is displayed by a display device (8).

11. Data processing system (7, 9) adapted to carry out a computer-implemented method according to one of claims 1 to 9.

12. X-ray imaging system (1) comprising
- an X-ray source (3), an X-ray detector (4), and a control system (7), which is configured to control the X-ray source (3) and the X-ray detector (4) to generate an X-ray image (13);
- a data processing system (7, 9), which is adapted to carry out a computer-implemented method according to one of claims 8 or 9; and
- a display device (8), wherein the control system (7) is configured to control the display device (8) to display the processed X-ray image (12, 21) or an image depending on the processed X-ray image (12, 21).

13. Computer program product comprising
- instructions, which, when executed by a data processing system (7, 9), cause the data processing system (7, 9) to carry out a computer-implemented method according to one of claims 1 to 9; and/or
- further instructions, which, when executed by an X-ray imaging system (1) according to claim 12, cause the X-ray imaging system (1) to carry out an X-ray imaging method according to claim 10.

## Patentansprüche

1. Computerimplementiertes Verfahren zum Erzeugen eines rauschvarianzstabilisierten Röntgenbildes (18), wobei
- ein Röntgenbild (13), erzeugt durch ein Röntgenbildgebungssystem (1), und ein entsprechender Satz von Bildgebungsparametern (14) des Röntgenbildgebungssystems (1) empfangen wird, wobei der Satz von Bildgebungsparametern (14) umfasst
- eine Spitzenspannung der Röntgenquelle (3); und
- einen Röhrenstrom der Röntgenquelle (3); und
- eine Röntgenimpulsdauer;
**dadurch gekennzeichnet, dass**
- ein Rauschpegelparameter (16) berechnet wird durch Simulieren einer Energiedeposition von Röntgenquanten, die von einer Röntgenquelle (3) des Röntgenbildgebungssystems (1) emittiert werden, auf einen Röntgendetektor (4) des Röntgenbildgebungssystems (1) abhängig von dem Satz von Bildgebungsparametern (14),
wobei der Rauschpegelparameter (16) abhängig von der simulierten Energiedeposition unter Verwendung eines Detektormodells für den Röntgendetektor (4), das eine Umwandlung von Röntgenquanten in optische Photonen und eine Umwandlung der optischen Photonen in ein elektrisches Detektorsignal modelliert, berechnet wird; und
- das rauschvarianzstabilisierte Röntgenbild (18) durch Anwenden einer varianzstabilisierenden Transformation, die von dem Rauschpegelparameter abhängt, auf das Röntgenbild (13) erzeugt wird.

2. Computerimplementiertes Verfahren nach dem vorstehenden Anspruch, wobei der Satz von Bildgebungsparametern (14) umfasst
- ein Filtermaterial und eine Filterdicke eines Röntgenfilters (22) des Röntgenbildgebungssystems (1); oder
- eine Kollimatoröffnungsgröße eines Röntgenkollimators (23) des Röntgenbildgebungssystems (1); oder
- ein Filtermaterial und eine Filterdicke eines Röntgenfilters (22) des Röntgenbildgebungssystems (1) und eine Kollimatoröffnungsgröße eines Röntgenkollimators (23) des Röntgenbildgebungssystems (1).

3. Computerimplementiertes Verfahren nach einem der vorstehenden Ansprüche, wobei der Satz von Bildgebungsparametern (14) umfasst
- eine Position oder Ausrichtung der Röntgenquelle (3); und
- eine Position oder Ausrichtung des Röntgendetektors (4); oder
- eine Position oder Winkelstellung eines C-Arms (2), der die Röntgenquelle (3) und den Röntgendetektor (4) trägt; und/oder
- eine Position und/oder Ausrichtung eines Patiententischs (5) oder eines Teils des Patiententischs (5); und/oder
- eine effektive Dicke eines abgebildeten Objekts (6).

4. Computerimplementiertes Verfahren nach einem der vorstehenden Ansprüche, wobei der Satz von Bildgebungsparametern (14) umfasst
- einen Verstärkungsfaktor des Röntgendetektors (4); oder
- einen Statusparameter eines Streuschutznetzes (24) des Röntgenbildgebungssystems (1); oder
- einen Pixel-Binning-Parameter des Röntgendetektors (4).

5. Computerimplementiertes Verfahren nach einem der vorstehenden Ansprüche, wobei die varianzstabilisierende Transformation eine generalisierte Anscombe-Transformation ist.

6. Computerimplementiertes Verfahren nach einem der vorstehenden Ansprüche, wobei eine Monte-Carlo-Simulation verwendet wird oder eine Sequenz von Monte-Carlo-Simulationen verwendet wird, um die Energiedeposition zu simulieren, wobei die Simulation der Energiedeposition offline durchgeführt wird und das Ergebnis der Simulation in einer Nachschlagetabelle gespeichert wird, die in Echtzeit ausgelesen wird.

7. Computerimplementiertes Verfahren nach einem der vorstehenden Ansprüche, wobei Simulieren der Energiedeposition umfasst
- Simulieren einer Kollimation durch einen Röntgenkollimator des Röntgenbildgebungssystems (1); oder
- Simulieren einer Röntgenabschwächung oder Röntgenstreuung oder Röntgentransmission durch das abgebildete Objekt (6) oder ein oder mehrere weitere Objekte.

8. Computerimplementiertes Verfahren zum Verarbeiten eines Röntgenbildes (13), wobei ein rauschvarianzstabilisiertes Röntgenbild (18) unter Verwendung eines computerimplementierten Verfahrens nach einem der vorstehenden Ansprüche und erzeugt wird, und
- ein Entrauschungsalgorithmus (19) auf das rauschvarianzstabilisierte Röntgenbild (18) angewendet wird; oder
- ein Bildverarbeitungsalgorithmus auf das rauschvarianzstabilisierte Röntgenbild (18) angewendet wird.

9. Computerimplementiertes Verfahren nach Anspruch 8, wobei Anwenden des Entrauschungsalgorithmus oder des Bildverarbeitungsalgorithmus Anwenden eines trainierten Machine-Learniung-Modells (19) auf das rauschvarianzstabilisierte Röntgenbild (18) umfasst.

10. Röntgenbildgebungsverfahren, wobei ein Röntgenbild (13) durch ein Röntgenbildgebungssystem (1) erzeugt wird und ein computerimplementiertes Verfahren nach einem der Ansprüche 8 oder 9 basierend auf dem Röntgenbild (13) durchgeführt wird und das verarbeitete Röntgenbild (12, 21) oder ein Bild, das von dem verarbeiteten Röntgenbild (12, 21) abhängt, durch eine Anzeigevorrichtung (8) angezeigt wird.

11. Datenverarbeitungssystem (7, 9), ausgelegt zum Durchführen eines computerimplementierten Verfahrens nach einem der Ansprüche 1 bis 9.

12. Röntgenbildgebungssystem (1) umfassend:
- eine Röntgenquelle (3), einen Röntgendetektor (4) und ein Steuersystem (7), das dazu ausgelegt ist, die Röntgenquelle (3) und den Röntgendetektor (4) zu steuern, um ein Röntgenbild (13) zu erzeugen;
- ein Datenverarbeitungssystem (7, 9), ausgelegt zum Durchführen eines computerimplementierten Verfahrens nach einem der Ansprüche 8 bis 9; und/oder
- eine Anzeigevorrichtung (8), wobei das Steuersystem (7) dazu ausgelegt ist, die Anzeigevorrichtung (8) zu steuern, um das verarbeitete Röntgenbild (12, 21) oder ein Bild, das von dem verarbeiteten Röntgenbild (12, 21) abhängt, anzuzeigen.

13. Computerprogrammprodukt umfassend
- Anweisungen, die bei Ausführung durch ein Datenverarbeitungssystem (7, 9) das Datenverarbeitungssystem (7, 9) veranlassen, ein computerimplementiertes Verfahren nach einem der Ansprüche 1 bis 9 auszuführen; und/oder
- weitere Anweisungen, die bei Ausführung durch ein Röntgenbildgebungssystem (1) nach Anspruch 12 das Röntgenbildgebungssystem (1) veranlassen, ein Röntgenbildgebungsverfahren nach Anspruch 10 auszuführen.

## Revendications

1. Procédé mis en œuvre par ordinateur pour créer une image (18) de rayons X stabilisée en variance de bruit, dans lequel
- une image (13) de rayons X créée par un système (1) d'imagerie par rayons X et un ensemble correspondant de paramètres (14) d'imagerie du système (1) par rayons X sont reçus, dans lequel l'ensemble de paramètres (14) d'imagerie comprend
- un kilovoltage pic de la source (3) de rayons X ; et
- un courant de tube de la source (3) de rayons X ; et
- une durée d'impulsion des rayons X ;
**caractérisé en ce qu'**
- un paramètre (16) de niveau de bruit est calculé en simulant un dépôt d'énergie de quanta de rayons X émis par la source (3) de rayons X du système (1) d'imagerie par rayons X sur un détecteur (4) de rayons X du système (1) d'imagerie par rayons X en fonction de l'ensemble de paramètres (14) d'imagerie,
dans lequel le paramètre (16) de niveau de bruit est calculé en fonction du dépôt d'énergie simulé en utilisant un modèle de détecteur pour le détecteur (4) de rayons X, qui modélise une conversion de quanta de rayons X en des photons optiques et une conversion des photons optiques en un signal électrique de détecteur ; et
- l'image (18) de rayons X stabilisée en variance du bruit est créée en appliquant à l'image (13) de rayons X une transformation stabilisant la variance, qui dépend du paramètre de niveau de bruit.

2. Procédé mis en œuvre par ordinateur suivant la revendication précédente, dans lequel l'ensemble de paramètres (14) d'imagerie comprend
- un matériau de filtre et une épaisseur de filtre d'un filtre (22) de rayons X du système (1) d'imagerie par rayons X ; ou
- une dimension d'ouverture de collimateur d'un collimateur (23) de rayons X du système (1) d'imagerie par rayons X ; ou
- un matériau de filtre et une épaisseur de filtre d'un filtre (22) de rayons X du système (1) d'imagerie par rayons X et une dimension d'ouverture de collimateur d'un collimateur (23) de rayons X du système (1) d'imagerie par rayons X.

3. Procédé mis en œuvre par ordinateur suivant l'une des revendications précédentes, dans lequel l'ensemble de paramètres (14) d'imagerie comprend
- une position ou une orientation de la source (3) de rayons X ; et
- une position ou une orientation du détecteur (4) de rayons X ; ou
- une position ou une angulation d'un bras (2) en C portant la source (3) de rayons X et le détecteur (4) de rayons X ; et/ou
- une position et/ou une orientation d'une couchette (5) de patient ou d'une partie de la couchette (5) du patient ; et/ou
- une épaisseur effective d'un objet (6), dont on a pris une image.

4. Procédé mis en œuvre par ordinateur suivant l'une des revendications précédentes, dans lequel l'ensemble de paramètres (14) d'imagerie comprend
- un facteur de gain du détecteur (4) de rayons X ; ou
- un paramètre de statut d'une grille (24) d'anti-dispersion du système (1) d'imagerie par rayons X ; ou
- un facteur de regroupement de pixels du détecteur (4) de rayons X.

5. Procédé mis en œuvre par ordinateur suivant l'une des revendications précédentes, dans lequel la transformation stabilisant la variance est une transformation d'Anscombe généralisée.

6. Procédé mis en œuvre par ordinateur suivant l'une des revendications précédentes, dans lequel on utilise une simulation de Monte-Carlo ou une séquence de simulation de Monte-Carlo pour simuler le dépôt d'énergie, dans lequel la simulation du dépôt d'énergie est effectuée hors ligne et le résultat de la simulation est mis en mémoire dans une table de consultation, qui est lue en temps réel.

7. Procédé mis en œuvre par ordinateur suivant l'une des revendications précédentes, dans lequel simuler le dépôt d'énergie comprend
- simuler une collimation par un collimateur de rayons X du système (1) d'imagerie par rayons X ; ou
- simuler une atténuation de rayons X ou une dispersion de rayons X ou une transmission de rayons X par l'objet (6), dont on a pris une image ou par un ou plusieurs autres objets.

8. Procédé mis en œuvre par ordinateur de traitement d'une image (13) de rayons X, dans lequel une image (18) de rayons X stabilisée en variance de bruit est créée en utilisant un procédé mis en œuvre par ordinateur suivant l'une des revendications précédentes, et
- un algorithme (19) de débruitage est appliqué à l'image (18) de rayons X stabilisée en variance de bruit ; ou
- un algorithme de traitement d'image est appliqué à l'image (18) de rayons X stabilisée en variance de bruit.

9. Procédé mis en œuvre par ordinateur suivant la revendication 8, dans lequel appliquer l'algorithme de débruitage ou l'algorithme de traitement d'image comprend appliquer un modèle (19) d'apprentissage automatique ayant subi un apprentissage à l'image (18) de rayons X stabilisée en variance de bruit.

10. Procédé d'imagerie par rayons X, dans lequel une image (13) de rayons X est créée par un système (1) d'imagerie par rayons X, et un procédé mis en œuvre par ordinateur suivant l'une des revendications 8 ou 9 est effectué sur la base de l'image (13) de rayons X, et l'image (12, 21) de rayons X traitée ou une image dépendant de l'image (12, 21) de rayons X traitée est affichée par un dispositif (8) d'affichage.

11. Système (7, 9) de traitement de données propre à effectuer un procédé mis en œuvre par ordinateur suivant l'une des revendications 1 à 9.

12. Système (1) d'imagerie par rayons X comprenant
- une source (3) de rayons X, un détecteur (4) de rayons X et un système (7) de commande, qui est configuré pour commander la source (3) de rayons X et le détecteur (4) de rayons X, afin de créer une image (13) de rayons X ;
- un système (7, 9) de traitement de données, qui est propre à effectuer un procédé mis en œuvre par ordinateur suivant l'une des revendications 8 ou 9 ; et
- un dispositif (8) d'affichage, dans lequel le système (7) de commande est configuré pour commander le dispositif (8) d'affichage, afin d'afficher l'image (12, 21) de rayons X traitée ou une image, qui dépend de l'image (12, 21) de rayons X traitée.

13. Produit de programme d'ordinateur comprenant
- des instructions, qui, lorsqu'elles sont exécutées par un système (7, 9) de traitement de données, font que le système (7, 9) de traitement de données exécute un procédé mis en œuvre par ordinateur suivant l'une des revendications 1 à 9 ; et/ou
- d'autres instructions, qui, lorsqu'elles sont exécutées par un système (1) d'imagerie par rayons X suivant la revendication 12, font que le système (1) d'imagerie par rayons X exécute un procédé d'imagerie par rayons X suivant la revendication 10.
